# EUROPEAN PATENT APPLICATION

(11) **EP 4 656 228 A1**
(43) Date of publication of application: **03.12.2025**
(21) Application number: 24179214.2
(22) Date of filing: 31.05.2024
(51) Int. Cl.: A61M 25/00, A61B 1/018

(54) **SMALL MULTI-LUMEN ENDOSCOPIC SYSTEM**

(71) Applicant: Lys Medical SA, 6041 Charleroi (BE)
(72) Inventor: Blanc, Loïc, 6041 Charleroi (BE); Geuens, Nicolas, 6041 Charleroi (BE)
(74) Representative: Icosa Europe

(57) **Abstract**

The present invention relates to a catheter (100), said catheter comprising a catheter body and at least two lumens configured for passing of a tool (50) wherein a natural section of the first lumen is smaller than a head section of the tool (50), the first lumen being configured to reach a tool insertion state.

## Description

### FIELD OF INVENTION

The present invention relates to a multi-lumen catheter having a reduced size configured to receive large sized tool. The invention also relates to an endoscopic system wherein tools are inserted into the multi-lumen catheter.

### BACKGROUND OF INVENTION

In current endoscopic procedures, endoscopes comprising a camera are used for imaging or treating intrabody parts of a human or animal subject through minimally invasive procedure. Imaging allows to precisely position the endoscope and thus the endoscopic probe at a target zone to operate different tools such as biopsy or ablation tools on the target zone.

However, the known endoscopes are not satisfactory. Indeed, in most endoscopes, the camera is permanently fixed in the operating channel of the outer wall of the endoscope. Therefore, these endoscopes have a diameter that is larger than the sum of the size of the camera and the endoscopic tool. Consequently, as the camera is fixed in the endoscope, the endoscopic tool passing in the operating channel has a limited size. Moreover, Consequently, smaller endoscopes are required for smaller internal diameter target zone, limiting the size of the endoscopic tool. Moreover, Only one tool can be inserted in the operating channel, if two very small tools are inserted, they could entangle. One solution is to increase the accessible size for the endoscopic tool or to increase the operating channel size. For example, the endoscope comprising the camera is first inserted in an orifice of the subject to reach the target zone. The camera is then removed from the endoscope and replaced by the appropriate tool, for example a biopsy tool or an ablation tool. This solution is not satisfactory because the biopsy or ablation tool is thus manipulated without direct imaging. Another solution is to reduce the size of the tools. However, reducing the size of the camera leads to a reduction of the image quality and reducing the size of the other tool such as the biopsy tool increases the risk of wrong use of the tool such as the extraction of too little tissue samples or even no tissue at all.

A second category of endoscopes are multi-lumen endoscopes that allow to simultaneously receive several tools, for example, a camera and at least one operating tool, with one tool in each lumen. However, these endoscopes are not satisfactory. Indeed, the size of the multi-lumen endoscopes is limited by the size of the natural orifice. However, the endoscope should be large enough to allow all the tools to be inserted. Therefore, the size of the endoscope is also limited by the total size of the tools.

Finally, endoscopes are expensive devices which have only several standard sizes. Therefore, the same endoscope is used for several combinations of camera and endoscopic tool even leading to a size of endoscope which is generally not adapted.

There is thus a need for an endoscopic system compatible with the current endoscopes and which may be inserted into very small orifices while allowing a direct imaging of the operation of the surgical tools with high quality.

### SUMMARY

This invention thus relates to a catheter, said catheter comprising a catheter body defining a catheter axis and comprising at least a first lumen and a second lumen adjacent to the first lumen, each lumen extending along the catheter axis, each lumen having a natural section, each lumen being configured to allow passage of a tool along the catheter axis, the tool comprising a head part with a head section,
wherein the natural section of the first lumen is smaller than the head section of the tool intended to pass in said first lumen, the first lumen being configured to reach a tool insertion state, the first lumen comprising, in the tool insertion state, an insertion part configured to receive the head part of said tool, the insertion part comprising the natural section of the first lumen and at least part of a natural section of the second lumen, the insertion part extending locally along the catheter axis, the insertion part having a variable position along the catheter axis to allow the passage of the head part along the catheter axis.

In other words, the invention relates to a multi-lumen catheter that allows insertion of tools that are larger than the size of the lumens in their resting undeformed states. To that end, the tool may encroach upon the section of other lumens during its insertion without increasing the external size of the catheter.

The catheter is cheaper to manufacture than the endoscope. Therefore, the size of the catheter may be chosen according to the combination of the camera and endoscopic tool which is used. The known endoscopes may be used to insert the catheter in the beginning of the orifice while the catheter may be guided up to the small target zone.

The catheter of the disclosure may therefore be inserted up to very small target zones while still allowing a direct imaging of the operation of the surgical tools with high quality thanks to a camera inserted in the catheter and by further allowing passage of tools with a size sufficient to perform a satisfactory medical act.

Moreover, the tool insertion state allows multiple tools to be inserted sequentially (e.g. a second tool may be inserted while a first tool is already inserted in the catheter) since the lumens are able to reach the tool insertion state at different positions along the catheter axis. Therefore, the encroach of the tool upon the section of other lumens is local so that, at any position along the catheter axis, the sum of the size of the sections of the tools is lower than the section of the catheter. When a tool is moved along the catheter axis, the insertion part follows the tool progression. Thanks to the catheter of this disclosure, the external diameter of the catheter remains constant during the insertion of the tools. Therefore, in a peripherical lung biopsy, a large imaging device and a large biopsy tool may be sequentially inserted in the catheter to provide high quality imaging of the biopsy and therefore a high quality of biopsy.

The catheter of the disclosure may thus be used in a common endoscope to perform endoscopic operations in different fields such as bronchoscopy, colonoscopy, urology, endoscopic retrograde cholangiopancreatography (ERCP)...

According to an embodiment, the catheter body is configured to be inserted into a channel of a medical device for:
- endoscopy of natural orifices, the endoscopy being preferably one of bronchoscopy, gastroscopy, gastro-enterology, colonoscopy, endoscopic retrograde cholangiopancreatography, urology, or ear, nose and throat endoscopy,
- laparoscopy, or
- cardiology.

According to an embodiment, the catheter body has an external catheter sectional size lower than 5 mm, preferably ranging between 1.2 mm and 3 mm, and more preferably between 1.8 mm and 2.8 mm.

According to an embodiment, the adjacent lumens are superposed along a first axis perpendicular to the catheter axis, at least one auxiliary lumen being positioned along a second axis perpendicular to the first axis and perpendicular to the catheter axis

This superposition of the lumens along a first axis allows to provide lumens with a section as large as possible leading to a thickness of the external wall of the catheter along the first axis as small as possible. This also allows to provide, in a direction perpendicular to the first axis, a larger thickness of the external wall of the catheter. This larger thickness allows to create auxiliary lumens having preferably sections smaller than the other lumens. These auxiliary lumens allow to insert wires for angling the tip of the catheter (steerability). These auxiliary lumens may also host a lighting device such as a LED so as to improve the quality of the image received from the viewing angle of the imaging device inserted in one of the lumens. The auxiliary lumens may also be used for flushing.

According to a first configuration, the first lumen and the second lumen are separated by a deformable wall, the first lumen and the second lumen comprising a locally variable section between the natural section, a reduced section smaller than the natural section and an insertion section larger than the natural section, the deformable wall comprising an undeformed configuration and a deformed configuration, wherein the undeformed configuration defines the natural section of the first lumen and the second lumen, wherein the insertion part of the first lumen comprises the deformed configuration of the deformable wall, the first lumen thereby having the insertion section, the second lumen having the reduced section at a position of the insertion part along the catheter axis.

The deformable wall allows to accommodate the section of the lumen to the size of the tool. Therefore, tools of different shapes and different size may be inserted.

According to an embodiment of the first configuration, the deformable wall comprises at least one flap, each flap comprising a first side fixed to an outer wall of the catheter body and a second side opposite the first side, a deformation of the deformable wall between the undeformed configuration and the deformed configuration being a variation of the position of the second side relatively to the outer wall of the catheter body.

According to an embodiment of the first configuration, the deformable wall is a flexible wall, the flexible wall comprising two opposite sides, each opposite side being fixed to an outer wall of the catheter body along a fixation zone, a dimension of flexible wall between the two opposite sides in the undeformed configuration being larger than a minimum distance between the two fixation zones leading to a loosen wall, the flexible wall being unfolded in the deformed configuration.

This allows to prevent any leaking between the two adjacent lumens. Moreover, thanks to the oversizing of the flexible wall compared to the size of the catheter, very small force could be needed to unfold the flexible wall during the passage of the tool. The deformation of the lumen is not due to a change in length of the membrane (by elasticity for instance), but just replacing the membrane in the best configuration to let the tool pass through.

According to an embodiment of the first configuration, the deformable wall is an elastic wall, the elastic wall comprising two fixation sides, each opposite side being fixed to an outer wall of the catheter body along a fixation zone, a dimension of elastic wall between the two fixation sides in the undeformed configuration being equal to a minimum distance between the two fixation zones, the elastic wall being stretched in the deformed configuration.

This allows to prevent any leaking between the two adjacent lumens. Moreover, thanks to elastic behavior of the elastic wall, the deformable wall recovers its undeformed shape after the passage of the tool.

According to an embodiment of the first configuration, the deformable wall is made of metal or polymer, preferably thermoplastics, elastomers, thermosets, thermoplastic elastomer, polyamide or fluoropolymer.

Thermoplastics, elastomers, thermosets have very good elastic properties. Polymers have advantageous elastic properties compared to metals as for smaller forces, they present larger deformation. Furthermore, in general, their limit of deformation (before plasticity) is larger than the range in metals, allowing for larger local deformation with no damage or permanent deformation of the structure. Polyamide and thermoplastics or thermoplastic elastomers can advantageously be extruded to easily manufacture the catheter. Fluoropolymer may for instance be shaped with a very small thickness which is advantageous when manufacturing the deformable wall. Fluoropolymers also present the advantage of having a very low friction coefficient, making sure that the tools could slide easily in the catheter. Furthermore, fluoropolymer could be etched for a good adhesion on other polymers for the assembly, while where the polymer is not etched, the other polymer will not adhere, ensuring that the lumens could be obtained. A very thin metallic structure could be deformed with low forces, and would ease the manufacturing of such catheter. Superelastic metals (such as Nitinol) could also be advantageous.

According to an embodiment of the first configuration, the deformable wall has a thickness ranging from 0.01 mm to 1 mm, preferably ranging from 0.025 mm to 0.1 mm.

This allows to separate the lumens and thus the tools with a minimum increase of the total diameter of the catheter.

According to a second configuration, the first lumen and the second lumen are joined along an interfacing opening thereby forming a merged lumen along the catheter axis, a union of the natural section of the first lumen and the natural section of the second lumen forming a merged section, the insertion part of the first lumen defining an insertion section comprising at least part of the merged section larger than the natural section of said first lumen, the head part passing in the first lumen and the second lumen.

This allows to separate the tools and thus avoids intermingling of the tools even if the lumens are joined without physical separation, since each lumen serves as a guide for the tool passage. This second configuration avoids the use of a separation wall thereby reducing the total diameter of the catheter or increasing the size of the tools to be passed without increasing the size of the catheter. This could also simplify the manufacturing as no very thin wall need to be assembled/extruded in the catheter. The shape could be more complex as it could be directly extruded. For the assembly, specific mandrels could be required. Metallic tubing with punched structure could form the shape to separate the tool passages.

According to an embodiment, the catheter is coiled, braided and/or made of metal.

This avoids kinking while bending the catheter. The reinforcement of the catheter is essential to provide advanced mechanical performance to the catheter made of polymer. When only polymer is used, the catheter strength and mechanical performance are limited. The reinforcement (braiding or coiling) improves the kink resistance, the pushability and torquability. As the catheter is used to provide a support for endoscopic tools and should slide in endoscope and/or in natural orifices of a patient, the force and displacement/rotation applied on the proximal side should be transmitted to the distal side. If a metallic tube is used, it may be too rigid. Nitinol and other superelastic materials provide interesting mechanical properties for such catheter. Other metallic tubing could be cut (for instance with laser cutting) to gives custom specific mechanical properties as lower rigidity and better flexibility. Furthermore, depending on the cut size and shape, the range of acceptable deformation could be adjusted (thanks to mechanical stops). The tube could be made of an assembly of numerous separated elements (such as beads or vertebrae) providing very flexible (or adjustable stiffness) structures.

According to an embodiment, a thickness of an outer wall of the catheter body is range from 0.025 mm to 3 mm, preferably from 0.05 mm to 1 mm, even more preferably from 0.05 mm to 0.5 mm.

Large thickness of the outer wall allows to insert, inside the thickness of the outer wall, bending wires or electrical wires powering a lighting device disposed at the tip of the catheter. When the thickness of the wall is larger, the reinforcement could be improved. Larger walls are not easily available in reduced size catheter in the medical field. A thin wall metallic tube could help in decreasing the overall wall thickness. If braiding or coiling is used, an outer layer is required to keep the reinforcement in place, to avoid that the metallic structure is exposed to the environment (contact with the tissues of the patient or the inner wall of the endoscope operating channel).

The invention also relates to a medical system comprising:
- A medical device comprising an operating channel;
- a catheter, said catheter comprising a catheter body defining a catheter axis and comprising at least a first lumen and a second lumen adjacent to the first lumen, each lumen extending along the catheter axis, each lumen having a natural section, each lumen being configured for passing a tool along the catheter axis, the catheter body being inserted into the operating channel of the medical device; and
- at least one tool, each tool comprising a head part with a head section, each tool being received in one of the lumens of the catheter body;
wherein the head section of a first tool of the at least one tool is larger than the natural section of the first lumen, the first lumen being configured to reach a tool insertion state, the first lumen comprising, in the tool insertion state, an insertion part configured to receive the head part of the first tool, the insertion part comprising the natural section of the first lumen and at least part of a natural section of the second lumen, the insertion part extending locally along the catheter axis, the insertion part having a variable position along the catheter axis to allow the passage of the head part of the first tool along the catheter axis.

According to an embodiment, a first tool of the at least one tool further comprises a body part having a body section, the body section being smaller than or equal to the natural section of the first lumen and wherein the head section of a second tool of the at least one tool is larger than the natural section of the second lumen, the second lumen being configured to reach a tool insertion state, the second lumen comprising, in the tool insertion state, an insertion part configured to receive the head part of the second tool, the insertion part comprising the natural section of the second lumen and at least part of a natural section of the first lumen, the insertion part extending locally along the catheter axis, the insertion part having a variable position along the catheter axis to allow the passage of the head part along of the second tool the catheter axis following the passage of the first tool.

According to an embodiment, at least one of the tools is an imaging device, the medical system thereby forming and endoscope.

This endoscopic system thus allows to provide direct imaging of the operation of the second tool. Having a larger head of the imaging device is useful for this invention (to have an insertion part moving along the length of the catheter), as the imaging sensor is usually taking additional space compared to the cable to transmit energy and data.

According to an embodiment, the head section of the first tool is larger than or equal to 0.5 mm and the head section of the second tool is larger than 1 mm, a union of the natural section of the first lumen and the second lumen being smaller than a sum of the two head section.

The invention also relates to a method for assembling a catheter system, the catheter system comprising a catheter, said catheter comprising a catheter body defining a catheter axis and comprising at least a first lumen and a second lumen adjacent to the first lumen, each lumen extending along the catheter axis, each lumen having a natural section, each lumen being configured for passing a tool along the catheter axis, wherein the first lumen and the second lumen are separated by a deformable wall, each of the first lumen and the second lumen comprising a locally variable section between the natural section, a reduced section smaller than the natural section and an insertion section larger than the natural section, wherein the deformable wall comprises an undeformed configuration and a deformed configuration, wherein the undeformed configuration defines the natural section of the first lumen and the second lumen, the catheter system further comprising at least two tools, each tool comprising a head part with a head section, the natural section of the first lumen being smaller than the head section of the tool intended to pass in said first lumen, said first lumen being configured to reach a tool insertion state, the catheter body comprising an input side and an output side, the method comprising:
- inserting sequentially the head part of each tool in one of the lumens through the input side, each lumen receiving at most one tool, the deformable wall of the first lumen being deformed to locally adopt, at a position of the head part along the catheter axis, the tool insertion state by increasing, at the position of the head part along the catheter axis, a section of the first lumen up to the insertion section comprising the natural section of the first lumen and at least part of a natural section of the second lumen thereby forming an insertion part extending locally along the catheter axis, a section of the second lumen being proportionally reduced down to the reduced section at the position of the head part along the catheter axis;
- sliding each tool along the lumens until the head part of the tools outputs from the output side, thereby shifting a position of the insertion part along the catheter axis.

The invention also relates to a method for assembling a catheter system, the catheter system comprising a catheter, said catheter comprising a catheter body defining a catheter axis and comprising at least a first lumen and a second lumens adjacent to the first lumen, each lumen extending along the catheter axis, each lumen having a natural section, each lumen being configured for the passage of a tool, wherein the first lumen and the second lumen are joined along an interfacing opening thereby forming a merged lumen along the catheter axis, the union of the natural section of the first lumen and the natural section of the second lumen forming a merged section, the catheter system further comprising at least two tools, each tool comprising a head part having a head section, , the head section of a first tool of said tools being larger than the natural section of the first lumen, the first lumen being configured to reach a tool insertion state, the first lumen comprising, in the tool insertion state, an insertion part configured to receive the head part of the tool, the insertion part defining an insertion section comprising at least part of the merged section larger than the natural section, the catheter body comprising an input side and an output side, the method comprising:
- inserting sequentially the head part of each tool in the catheter body through the input side, the head part of the first tool being inserted in the merged lumen, the first lumen thereby reaching the tool insertion state with the insertion part located at the location of the head part, the insertion section being equal to a sum of the natural section of the first lumen and a part of the natural section of the second lumen occupied by the head section;
- sliding each tool along the catheter axis until the head part of each tool completely outputs from the output side, thereby shifting the position of the insertion part along the catheter axis;
- placing each tool in one of the lumens, each lumen receiving at most one tool.

### DEFINITIONS

In the present invention, the following terms have the following meanings:
"Deformable" refers to the characteristic of the separating wall in the first configuration of the invention. The deformability allows a movement of the center of the separating wall along the direction perpendicular to its thickness of more than 100% of its thickness, preferably more than 150% of its thickness, when an external force is applied. The deformable wall recovers its resting or undeformed state when the external force disappears.
"Elastic" refers to the characteristic of the deformable wall which is able to be stretched upon application of an external force and to shrink when the external force is no longer applied. The elasticity allows an increase of the length of the deformable wall by more than 10% of its thickness, preferably more than 50% of its length when force is applied.
"Flexible" refers to the characteristic of an element which is able to bend or curves easily under an external force. The flexible element allows to modify the absolute distance between two points of said element of more than 10%, preferably more than 50% without changing the total size of the element.
"Natural section" refers to the section of the lumen when no tool is inserted in said lumen or in the adjacent lumen.
"Part" refers to a portion of an element (such as the catheter, the lumen or the tool) defined parallelly to the longitudinal axis of said element such as the catheter axis or the tool axis. The part of an element is thus a segment of this element.
"Section": refers to the shape of an element (such as the catheter, the lumen or the tool) in a plane perpendicular to the longitudinal axis of said element such as the catheter axis or the tool axis. The section includes the form but also the dimensions of the element measured perpendicularly to the longitudinal axis.
"Small orifices": refers to the orifices of the body of a subject having an internal diameter lower than 10 mm. The small orifices have a diameter larger than 0 mm, preferably larger than 5 mm. Conversely, large orifices are orifices of the body of a subject having an internal diameter larger than or equal to 10 mm. The small or large orifice may be a natural orifice (bronchial tubes, blood vessels, colon, duodenum, urethra...) or an incision.

### BRIEF DESCRIPTION OF THE DRAWINGS

**Figure 1** is a representation of the endoscopic system according to one embodiment of the invention.
**Figure 2** is a perspective and sectional representation of the catheter in one embodiment of the physically separated configuration.
**Figure 3** is a representation of a tool comprising a head part and a body part.
**Figure 4** is a perspective and sectional representation of the catheter of figure 2 in the tool insertion state, i.e., during the insertion of a tool.
**Figure 5** is a perspective and sectional representation of the catheter of figure 2 after the head part of the tool in completely output, the lumen recovers its rest state.
**Figure 6** is a perspective and sectional representation of the catheter of figure 2 wherein two tools are inserted, the sum of the head section of the two tools being larger than the sum of the natural sections of the lumens.
**Figure 7** is a combination of sectional views of a catheter in the first embodiment of the physically separated configuration. The sectional views showing from left to right: the catheter in rest state before the insertion of a tool, the tool insertion state of one lumen wherein a tool is inserted, the catheter in rest state after the head part of the tool is output, the catheter in rest state wherein the two lumens support a tool.
**Figure 8** is a combination of sectional views of a catheter in the second embodiment of the physically separated configuration. The sectional views showing from left to right: the catheter in rest state before the insertion of a tool, the tool insertion state of one lumen wherein a tool is inserted, the catheter in rest state after the head part of the tool is output, the catheter in rest state wherein the two lumens support a tool.
**Figure 9** is a combination of sectional views of a catheter in the third embodiment of the physically separated configuration. The sectional views showing from left to right: the catheter in rest state before the insertion of a tool, the tool insertion state of one lumen wherein a tool is inserted, the catheter in rest state after the head part of the tool is output, the catheter in rest state wherein the two lumens support a tool.
**Figure 10** is a combination of sectional views of a catheter in the second or third embodiment of the physically separated configuration wherein the deformable wall is made in one piece with the outer wall. The sectional views showing from left to right: the catheter in rest state before the insertion of a tool, the tool insertion state of one lumen wherein a tool is inserted, the catheter in rest state after the head part of the tool is output, the catheter in rest state wherein the two lumens support a tool.
**Figure 11** is a sectional view of a catheter in the second or third embodiment of the physically separated configuration wherein the fixation sides of the deformable wall are fixed to the same fixation zone.
**Figure 12** is a combination of sectional views of a catheter in the merged configuration. The sectional views showing from left to right: the catheter in rest state before the insertion of a tool, the tool insertion state of one lumen wherein a tool is inserted, the catheter in rest state after the head part of the tool is output, the catheter in rest state wherein the two lumens support a tool.
**Figure 13** is a sectional view of a catheter in the merged configuration comprising an auxiliary lumen.
**Figure 14** is a combination of longitudinal views of a catheter in the merged configuration. The top figure shows the tool insertion state of one of the lumens. The bottom view shows the catheter in the rest state after the head part of the tool is output.

### DETAILED DESCRIPTION

This invention relates to a medical system. The medical system may be used for endoscopy of small orifices of a subject in different fields such as bronchoscopy, gastroscopy, colonoscopy, urology, gastro-enterology, endoscopic retrograde cholangiopancreatography (ERCP), ear nose and throat (ENT) endoscopy, ... For example, the medical system of this disclosure advantageously allows to reach the periphery of the lung during a bronchoscopy wherein the bronchial tubes have an internal diameter lower than 3 mm, preferably lower than 2 mm. The medical system may also be used to perform laparoscopy or cardiology.

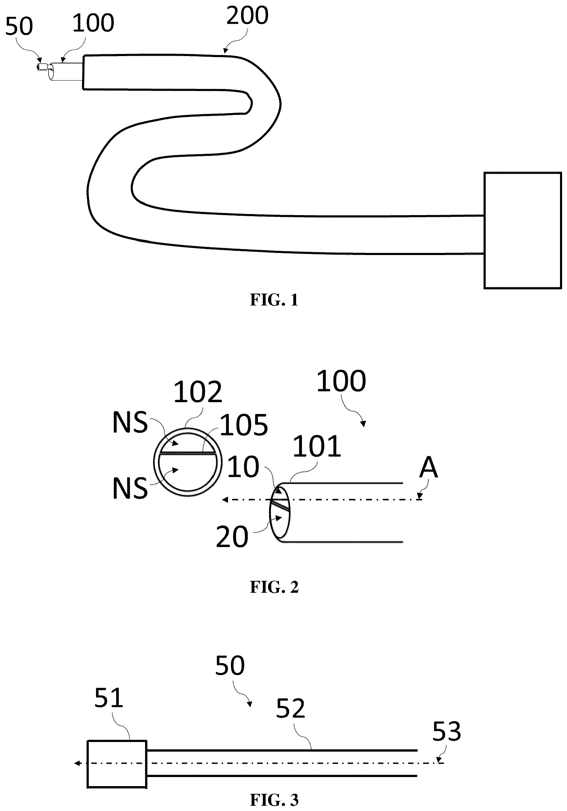

The medical system which is exemplarily represented in figure 1 comprises:
- a medical device 200;
- a catheter 100 configured to be inserted into the medical device 200; and
- at least one tool (50, 60), that may be an endoscopic probe, configured to be inserted in the catheter 100. During the insertion, the tool (50, 60) is supported or disposed in the catheter with a certain amount of freedom of movement allowing sliding (translation) and rotation.

Non-limitative examples of medical devices are cystoscope (for the bladder), nephroscope (for the kidney), bronchoscope (for the bronchus), arthroscope (for the joints), colonoscope (for the colon), or laparoscope (for the abdomen or pelvis). The medical device 200 may comprise at least one operating channel, preferably one channel, extending along a medical device axis. A length of the medical device 200 is thus measured along the medical device axis. Each operating channel is configured to receive a catheter, preferably a catheter 100 of this disclosure. The medical device 200 has a wall separating an interior of the catheter from an exterior of the medical device 200. The wall thus has an external surface and an internal surface. Each operating channel may have an internal size lower than 5 mm, preferably ranging between 1 mm and 3 mm, and more preferably between 1.2 mm and 2.8 mm. The internal size is a characteristic quantity measured between two points on the inside wall of the operating channel perpendicularly to the medical device axis (e.g. a diameter if the section of the operating channel is a circle, a length or a width if the section of the operating channel is a square, etc.). The operating channels preferably have a circular section, the internal size of the channel being its internal diameter. The medical device 200 may have an external size lower than 7 mm, preferably ranging between 3 mm and 5 mm. For gastroscopy, for example, the medical device 200 may have an external size larger than 7 mm. The external size is a characteristic quantity measured between two points on the external surface of the operating channel perpendicularly to the medical device axis (e.g. a diameter if the section of the operating channel is a circle, a length or a width if the section of the operating channel is a square, etc.). The medical device 200 preferably has a circular section, the external size of the medical device 200 being its external diameter. The medical device 200 further comprises an external surface that may be rendered biocompatible using a coating with a biocompatible material or using a biocompatible sheath disposed on the external surface, such as plastic.

The advantage of using a catheter 100 inserted in a medical device 200 is that the medical device 200 may have a single operating channel of large diameter wherein different catheters having different lumen shapes or sizes may be used according to the need of the operator. Furthermore, the medical device could be used as a support for the proximal part of the catheter that may be more flexible or fragile as designed to reach deeper location. It could also be used to provide an additional articulation/steering point for ease of navigation/orientation of the catheter. The catheter of the disclosure may thus be used in a common endoscope to perform endoscopic operations in different fields such as bronchoscopy, gastroscopy, colonoscopy, gastro-enterology, urology, endoscopic retrograde cholangiopancreatography (ERCP), ear nose and throat (ENT) endoscopy, ... The catheter of the disclosure may also be used in a common medical device comprising a channel to perform laparoscopy or cardiology.

The catheter 100 which is part of the invention, comprises a catheter body having a general shape of a tube with preferably a circular section. The dimensions of the section of the tube are small compared to its length. In other words, the catheter body extends along a catheter axis A. The catheter body thus has, by definition, two extremities: a proximal end (not shown) and a distal end 101. It will be appreciated that the terms "proximal" and "distal" are utilized herein with reference to the operator that manipulates the medical system. The proximal end may also be named the input side while the distal end 101 may also be named the output side.

The section of the catheter body is measured perpendicularly to the catheter axis A while the length of the catheter body is measured along the catheter axis A from the proximal end to the distal end 101. Preferably, the catheter 100 configured to be completely inserted into the medical device 200 while the extremities of the catheter 100 extend outside the medical device 200. Preferably, the section of the catheter body is constant along the catheter axis A. The definition of the catheter axis A does not imply that the catheter is rigid along the catheter axis A. Indeed, the catheter body is flexible along its length so as to be directed along an orifice of the subject.

The catheter body has an outer wall 102 separating an interior of the catheter from an exterior of the catheter. The outer wall 102 thus has an outer surface and an inner surface. The outer surface is adapted to be put in contact with the orifice of the subject. The outer surface is thus preferably biocompatible and smooth. The catheter body and more precisely its outer wall 102 may be coiled, braided and/or made of metal for reinforcement. To render the braided outer surface biocompatible, the outer surface may be coated with biocompatible material or a biocompatible sheath may be disposed on the outer surface, such as plastic. The outer layer may be made of polymer so as to keep a fixed structure in terms of braiding, but also to have better mechanical properties and surface finish of the catheter. The inner surface may also be coated with or made of slipping material to ease the insertion of the tools. The thickness of the coating or material is advantageously as thin as possible. For example, the thickness of the coating may be as small as 0.05 mm. The metallic outer wall 102 may comprise metallic sheets or tubes. Cuts may be disposed along the metallic sheets or tubes to provide required flexibility. Moreover, a coating or sheath may be applied on the metallic outer wall 102 to improve waterproofing of the catheter body. The catheter 100 can be made cost-effective for single use application. Advantageously, the outer wall 102 may be transparent, in order to allow optical visualization through said walls, for example, by an imaging device. This helps in getting better view and a protection again secretions, blood or to perform a check during the insertion of the probe. The whole catheter or just the distal end 101 of the catheter 100 may be transparent. The outer wall 102 may be made of, or comprises, a radio-opaque material to facilitate its localization, e.g. through external imaging techniques such as computer tomography or fluoroscopy.

The distal end 101 is advantageously atraumatic to prevent damaging tissue while navigating through an anatomical cavity. For example, the edge of the distal end 101 can be rounded and/or made of a softer, e.g. resilient, material. In some examples, the reinforcement braid in the wall of the catheter body extends distally from the distal tip, and the distal tip is advantageously free from reinforcement. A specific distal end could be designed for the catheter described in this disclosure to support the tools and to obtain atraumatic properties.

Advantageously, an actuation system, e.g. comprising pull cables or wires, may be inserted in the thickness of the outer wall 102 along the catheter axis A to make catheter body steerable. The actuation system allows to steer the distal end 101 from the proximal end.

Advantageously, the catheter body is configured to transmit torque between the proximal end and the distal end 101. An efficient torque transmission can be achieved by providing a suitable reinforcement braid in the outer wall 102. By so doing, a rotation about the catheter axis A effected at the proximal end can be transmitted to the distal end 101 to enable navigation within an anatomical cavity. It is additionally or alternatively possible to make the catheter body with a pre-formed shape, such as a bent, waved or other appropriate shape to ease navigation.

For example, the catheter 100 has an external catheter sectional size, i.e., the size of the section of the outer surface, lower than 5 mm, preferably ranging between 1.2 mm and 3 mm, and more preferably between 1.8 mm and 2.8 mm. The external catheter sectional size is a characteristic quantity measured between two points on the outer surface perpendicularly to the catheter axis (e.g. a diameter if the section of the catheter is a circle, a length or a width if the section of the catheter is a square, etc.). The catheter 100 preferably has a circular section, the external catheter sectional size being its external diameter. This advantageously allows to insert the catheter 100 in the medical device 200. Indeed, current medical devices, for example endoscopes, have an internal diameter lower than 5 mm. The smaller the external size of the catheter 100, the deeper through the orifices such as the bronchial tubes may be reached. The impact of the increase of the external size is even more important for small sized catheter. Indeed, an increase of external diameter of 0.3 mm on a catheter of 3 mm represents 10% of increase of the external diameter and 17% of increase of the sectional area of the catheter. The same difference on a catheter of 8 mm represents only 3% of increase of the external diameter and 7% of increase of the sectional area of the catheter. The small dimensions provide a catheter 100 which may be configured to reach small orifices, for example to reach a target zone at the periphery of a lung of a subject.

The catheter 100 comprises at least two lumens as represented for example in figure 2. The lumens are named the first lumen 10 and the second lumen 20 in the following. The first lumen 10 and second lumen 20 are interchangeable, the two lumens presenting the same properties. The catheter 100 may comprise additional lumens, such as a third lumen, a fourth lumen,... (not shown). The lumens (10, 20) are channels or extrusions formed in the catheter. The lumens (10, 20) comprise a length extending along the catheter axis A. The length of each lumen (10, 20) is equal to the length of the catheter body. Each lumen (10, 20) extends from an inlet aperture at the proximal end to an outlet aperture at the distal end 101. In other words, the inlet aperture and outlet apertures open onto the exterior of the catheter 100. Preferably, the lumens (10, 20) have circular sections but the section of the lumens may be shaped according to the shape of the tool to be inserted in or may have a shape maximizing the volume of the lumen (10, 20). The lumens, contrarily to auxiliary lumens defined hereafter, are thus configured to support the largest tools used for the medical operation. They are preferably disposed so as to maximize the section of the lumens in a given external catheter sectional size. The section of the lumens (10, 20) before tool insertion is named hereafter the natural section NS. It corresponds to the section of each lumen at rest or in the rest state, without being submitted to any external force other than the natural forces generated for example by gravity or by the catheter itself. The natural section NS of each lumen may be different. For example, the natural section NS is ranging between 0.2 mm and 2.6 mm, advantageously between 0.5 mm and 2 mm.

The inner surface of the catheter 100 thus corresponds to the total surface of the overall lumens (10, 20). The thickness of the outer wall 102 is thus defined by the distance between the inner surface and the outer surface. The thickness of an outer wall 102 of the catheter may be range from 0.025 mm to 1 mm, preferably from 0.05 mm to 0.3 mm, even more preferably from 0.05 mm to 0.1 mm. To reach such a small thickness, in case of braided outer wall 102, flat wires may be used. The flat wires may have a thickness as small as 0.025 mm. The wall thickness could be variable as explained hereafter.

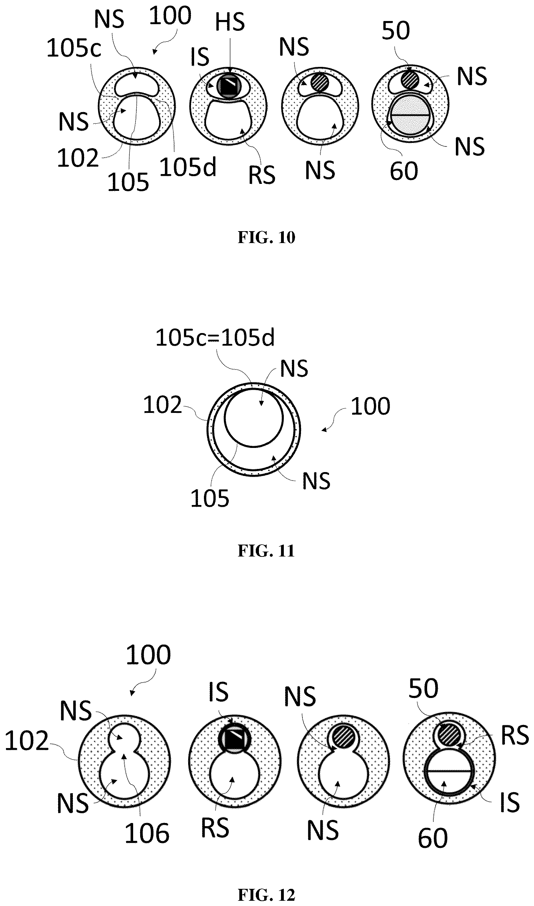

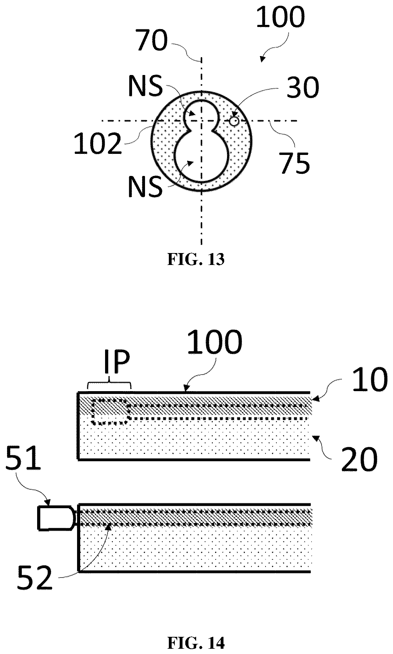

According to the shape of the lumens (10, 20), the thickness of the outer wall 102 may be different at different circumferential points as represented for example by the dotted areas in figures 10, 12 and 13. For example, in order to maximize the size of the natural section NS of the lumen, the lumens (10, 20) are superposed. In other words, the lumens (10, 20) are disposed along a first axis 70 perpendicular to the catheter axis A. Therefore, as represented in figure 13, the thickness of the outer wall 102 along this first axis 70 is small. However, along a second axis 75 perpendicular to the first axis 70 and perpendicular to the catheter axis A, the thickness of the outer wall 102 is larger than in the first axis 70. This allows to create at least one auxiliary lumen 30 along a second axis 75 as represented in figure 10. The auxiliary lumens 30 typically have a section smaller than the natural section of the other lumens (10, 20). These auxiliary lumens 30 are advantageous. Indeed, it allows to insert pull cables or wires, to make catheter body steerable without increasing the thickness of the outer wall 102. It also allows to insert a lighting device such as a LED or optical fibers so that to enlighten the viewing angle of the imaging device inserted in one of the lumens (10, 20). Finally, the auxiliary lumens 30 may also be used to flush the target zone with gas or fluid such as CO2, air, saline solution, physiological solution, water, or contrast agent.

At least the first lumen 10 and the second lumen 20 are adjacent. Adjacent lumens are lumens positioned close to each other, i.e., without any other lumen in between. For example, the lumens (10, 20) may be separated by a separating wall or may partially be merged without separating wall. There may be more than two adjacent lumens. For example, when three lumens are present in the catheter, the lumens may be adjacent two by two, the lumens being substantially superposed. In another example, each lumen is adjacent to the two other lumens, the lumens being disposed in a triangle.

Each lumen (10, 20) is configured for the passage of a tool (50, 60 along the catheter axis A, each tool being received in one of the lumens (10, 20). In other words, preferably no more than one tool is passed in a lumen. The tool (50, 60) is elongated along a tool axis 53. The tool 50 is preferably at least partially flexible, i.e., flexible along at least part of the tool axis 53. The tool (50, 60) has a length advantageously longer than a length of the catheter body allowing full insertion of said tool through the lumen of the catheter 100, and possibly deployment of one of the extremities of the tool (50, 60) beyond the outlet aperture of the lumen. The tool (50, 60) may have a constant section along its length. Alternatively, the tool 50 comprises a head part 51 and a body part 52, the body part 52 being connected to the head part 51 The head part 51 has a head section HS and a head length. The body part 52 has a body section smaller than the head section HS and a body length. The length of the tool (50, 60) is the sum of the length of the head part 51 and the body part 52. The head part 51 is preferably disposed at one extremity of the body part 52. Alternatively, the head part 51 is disposed between two portions of the body part 52, the two portions forming the body part 52. An example of tool comprising a head part 51 and the body part 52 is shown in figure 3. The tool 50 with a constant section may be seen as comprising a head part 51 and a body part 52 with a body section equal to the head section HS. The body section is preferably configured to be entirely disposed in the natural section NS of the lumen wherein the tool is inserted.

The tool (50,60) may be, for example, an imaging device, a lighting device, a biopsy device, a flushing device, a needle, a cryoprobe, a brush, an electromagnetic probe, a radial ultrasound probe, a therapeutic device such as an ablation probe,... For example, the smallest dimension of the head section HS may be larger than or equal to 0.5 mm, preferably larger than 1 mm. The impact of the increase of the size of the tool is very important for small sized catheter. Indeed, an increase of diameter of the head section HS of 0.1 mm on a tool of 1 mm represents, for a spherical head part 51, 10% of increase of the diameter and 25% of increase of the volume of the head part 51. The catheter 100 of this disclosure is thus particularly configured to adjust to the size variations of the tools without increasing the external size of the catheter 100. Since the tool is defined by its dimensions, the fluid or gas which may be injected in the lumen for, for example, flushing, is not considered as a tool in the meaning of this disclosure.

The imaging device may be a camera, preferably a CMOS (Complementary metal-oxide-semiconductor) camera, an ultrasound sensor, an echography device or a fiber bundle. The camera is preferred thanks to its better image quality. The medical device comprising the imaging device thus forms and endoscope. The known cameras have size which may be as small as 0.65 mm by side for an array of 400x400 pixels each having a size of 1 µm by side. The fiber bundle may comprise 100 000 fibers of 10 µm of diameter each. The imaging device preferably comprises a protective layer. The protective layer may have a thickness of more than 50 µm. The protective layer may be in plastic or metal. The advantage of using an imaging device in the catheter is that it allows frontal view contrarily to external devices such as ultrasound probes which offers a lateral view of the target zone. According to one embodiment, the medical system comprises tools, other than an imaging device, inserted in the lumen. In this embodiment, the imaging device may be included in the outer wall 102 of the catheter 100 or be part of the medical device 200, preferably in an undetachable manner. The imaging device is thus not part of the insertable tools (50, 60). In an alternative embodiment, the imaging device is part of the tools being passed in the lumens. The imaging device is thus reversibly disposed in the catheter so that it is re-usable which advantageously reduces the final cost of the system. A control unit can be comprised in the medical system. The control unit can comprise a visual display to display images taken by the imaging device.

The lighting device may be obtained by LED(s), preferably emitting white light. The LED is preferably as small as possible. The LED is preferably protected. The protective layer may be a transparent resin or a transparent window. The lighting device may be an optical fiber. The fiber may be integrated to the catheter braid. The lighting device may be included in the imaging device. Advantageously, the lighting device may be combined with the actuation system for steering the catheter.

The biopsy device may be a forceps which advantageously allows to obtain large size samples compared to a needle. Moreover, the forceps allows to maintain an intact cell structure for histological analysis and not only cytological analysis as in the case of a needle.

The catheter 100 of this disclosure advantageously allows the insertion of a tool (50, 60) having a head section HS larger than the natural section NS of the lumen (10, 20) without increasing the external diameter of the catheter body. The comparison between the sections of two elements is defined in this disclosure like this: a first section of a first element is larger than a second section of a second element if it does not exist an orientation of the first section relatively to the second section which allows the first section to be fully integrated into the second section. To allow the passage of large tools (e.g. having a section larger than the natural section NS), at least one of the lumens (10, 20) is configured to reach a tool insertion state allowing to proportionally vary its own section and the section of an adjacent lumen (20,10). In other words, in the tool insertion state, the length of the catheter body and the external diameter of the catheter 100 is not modified, only the sections of the lumens are modified.

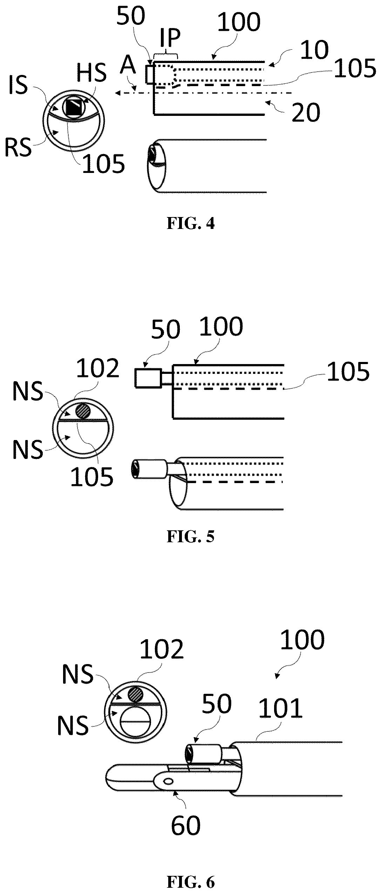

For example, considering the first lumen 10 configured to reach a tool insertion state and wherein a first tool 50 having a head section HS larger than the natural section NS of the first lumen is inserted, said first lumen 10 comprises, in the tool insertion state, an insertion part IP configured to receive the tool head of the first tool 50. The tool insertion state provides to the first lumen 10, at the position of insertion part IP, a section larger than the natural section 10. Proportionally, the section of the second lumen 20 adjacent to the first lumen 10 is reduced down to a reduced section RS. In other words, the insertion part IP has an insertion section IS that comprises the natural section NS of the first lumen 10 and at least part of a natural section NS of the second lumen 20. The tool insertion state is represented for example in figure 4 and figure 13. The insertion part IP has a length which extends along the catheter axis A, this length being conditioned by the widened portion of the tool, for example the head part 51. In other words, the insertion part IP is a part or a segment of the lumen. Therefore, the insertion part IP does not extend all along the catheter axis A. Indeed, the insertion part IP advantageously extends locally along the catheter axis A and the insertion part IP has a variable position along the catheter axis A to allow the progressive passage of the head part 51 from the inlet aperture to the outlet aperture. In other words, the insertion part IP is configured to progress along the catheter axis A to accommodate the head part 51 as it progresses along the catheter axis A. This advantageously allows to insert several tools (50, 60) sequentially without need to insert the whole length of a tool before inserting the other tool. This therefore reduces the duration of handling of the catheter 100 by a surgeon or a physician and thus reduces the duration of the medical procedure on a subject. In other words, the catheter 100 of this disclosure allows, for a union of the natural section of the first lumen 10 and the second lumen 20 smaller than a sum of the head section HS of two tools (50, 60), to insert these two said tools (50, 60) without increasing the external diameter of the catheter 100.

Explained differently, the tool insertion state is obtained by the insertion of the head part 51 of a tool 50 having a head section HS larger than the natural section NS of a lumen 10. Therefore, the length of the insertion part IP depends on the length of the head part 51 of the inserted tool 50 and the insertion section IS depends on the head section HS of the inserted tool 50.

The body section of the first tool 50 is smaller than or equal to the natural section NS of the first lumen 10. Therefore, when the head part 51 completely comes out from the lumen, the first lumen 10 recovers its natural section all along the catheter axis A as represented in figure 5. Having a body section smaller than the natural section NS of the first lumen 10 is advantageous because it allows the second lumen 20 to reach its tool insertion state when inserting a second tool 60 having a head section HS larger than the natural section of the second lumen 20 as represented in figures 6 and 12. Indeed, the insertion part IP of the second lumen 20 reaches an insertion section IS that comprises the natural section NS of the second lumen 20 and at least part of a natural section NS of the first lumen 10.

The volume of the first lumen 10 not occupied by the body part 52 may be used to inject flushing fluid such as liquid or gaz. To do so, a flushing valve may be connected to the inlet aperture of the lumen. Advantageously, to provide enough space for flushing, the lumen may have a sectional shape which does not correspond to the body section of the tool. For example, for a circular body section, the lumen may have a non-circular shape as represented in figures 5 and 10. Moreover, the second lumen 20 may be used to apply suction to remove the flushed fluid via the volume not occupied by the body part of the second tool 60 in the second lumen 20. The second lumen 20 may thus also have a sectional shape which does not correspond to the body section of the tool 60. Alternatively, the second lumen 20 may be used as a flushing lumen only when no tool is inserted.

A seal system may be advantageously provided at, or in connection with, the inlet aperture of at least one lumen (10, 20). The seal system comprises a sealing member configured to seal the lumen tightly to prevent fluid leakage when the tool (50, 60) is inserted therethrough. The sealing member can be formed of a soft or resilient, e.g. elastomeric, material. Sealing member can comprise a through-bore configured to slidingly receive the body part 52 of the tool.

At the proximal end, catheter 100 can comprise a connector module, e.g. comprising a screw connector or Luer-lock fitting, to fluid tightly connect the catheter 100 to a fluid delivery module in an advantageously releasable manner. A proximal portion of one of the lumens (10, 20) or auxiliary lumens 30 may extend through connector module.

### Physically separated configuration of the catheter 100

A first configuration of the catheter 100, also named the physically separated configuration, is illustrated in figures 2 and 4-9. In this first configuration, the first lumen 10 and the second lumen 20 are physically separated by a deformable wall 105. The deformable wall 105 is thus a layer or a membrane extending along the catheter axis A and separating the lumens. The deformable wall 105 advantageously helps to guide the tools. The deformable wall 105 is preferably gas tight and/or water tight thereby avoiding contamination between the two lumens. This advantageously allows to control a flow of fluid (gas or liquid) in each lumen independently. The deformable wall 105 may be made of super-elastic metals (such as Nitinol), metal, preferably very thin metallic structure, or polymer, preferably thermoplastics, elastomers, thermosets, polyamide or fluoropolymer.

The deformable wall 105 is deformed under a force generated substantially perpendicularly to the catheter axis A by the insertion of the head part 51 of a tool. The deformation of the deformable wall 105 allows to proportionally vary the section of the adjacent lumens (10, 20) respectively from the natural section NS to the insertion section IS and the reduced section RS at a given position along the catheter axis A. As explained above, the deformation of the deformable wall is local, i.e., extending over only a part of the catheter axis A.

The deformable wall 105 thus comprises an undeformed configuration wherein the deformable wall 105 is undeformed leading to lumens (10, 20) having their natural section NS all along the catheter axis A. The deformable wall 105 also has a deformed configuration wherein the deformable wall 105 is locally deformed, leading to lumens (10, 20) having respectively the insertion section IS and the reduced section RS over at least part of the catheter axis A. Therefore, in the tool insertion state of a lumen (10, 20), the part of the wall 105 which is deformed defines the insertion part IP.

To provide the deformability to the deformable wall 105, the deformable wall 105 preferably has a thickness (measured perpendicularly to the catheter axis A) ranging from 0.01 mm to 1 mm, preferably ranging from 0.025 mm to 0.1 mm.

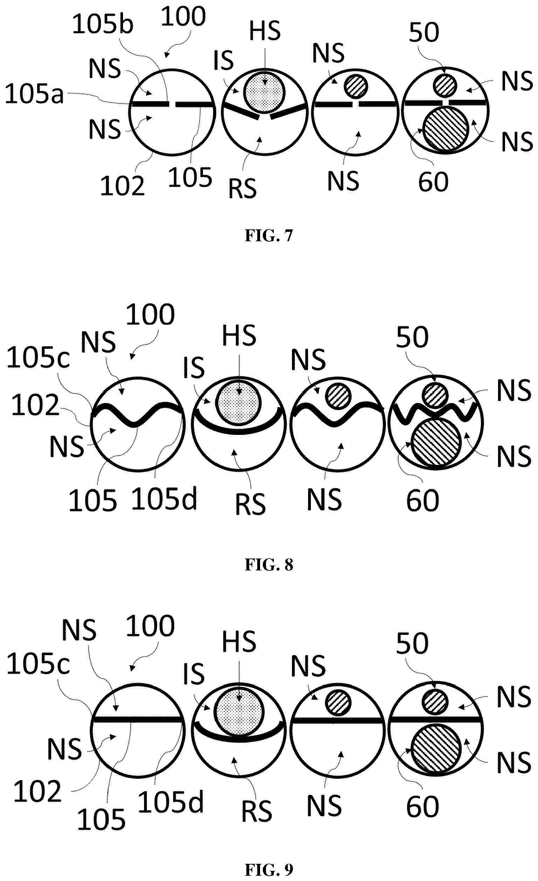

In a first embodiment of the physically separated configuration, the deformable wall 105 comprises at least one flap. Figure 7 shows an embodiment of the catheter 100 comprising two flaps. The flap comprises a first side 105a fixed to the outer wall 102 of the catheter body. This first side 105a is thus a longitudinal side extending preferably parallelly to the catheter axis A. The first side 105a is preferably fixed, for example by gluing, to the inner surface of the catheter. The flap may also be manufactured in one piece with the outer wall 102 of the catheter body. The flap further comprises a second side 105b opposite to the first side 105a. This second side 105b is thus also a longitudinal side extending preferably parallelly to the catheter axis A. The deformation of the deformable wall 105 corresponds to a variation of the position of the second side 105b relatively to the outer wall 102 of the catheter body. When the second side 105b is approaching the center of the lumen, this reduces the section of said lumen towards the reduced section RS. Inversely, the movement of the second side 105b away from the center of the lumen change said lumen towards the tool insertion state thereby increasing the section towards the insertion section IS.

The flap may be flexible or rigid. In the flexible flap, the variation of the position of the second side 105b relatively to the outer wall 102 is allowed thanks to the flexible behavior of the flap. In the rigid flap, the variation of the position of the second side 105b relatively to the outer wall 102 may be allowed by a rotational element, such as a hinge, fixed to the outer wall 102 and to the first side 105a of the flap leading to a rotation of the flap around the rotation axis of the rotational element.

In a second embodiment of the physically separated configuration, the deformable wall 105 is a flexible wall as illustrated in figure 8. The flexible wall comprises two fixation sides (105c, 105d), preferably, the longitudinal sides of the flexible wall extending parallelly to the catheter axis A. Each fixation side (105c, 105d) is fixed to the outer wall 102 of the catheter body along a fixation zone. In one embodiment, the two fixation sides (105c, 105d) are fixed along the same fixation zone as represented in figure 11. The two fixation sides (105c, 105d) thus coincide. The flexible wall may also be manufactured in one piece with the outer wall 102 of the catheter body. The fixation zone is therefore the contact zone between the outer wall 102 and the flexible wall. The flexible wall is flexible but not necessarily stretchable. Indeed, the flexible wall has a dimension measured perpendicular to the catheter axis, i.e. between the two opposite sides (105c, 105d) which is large compared to the distance between the two fixation zones, for example ranging between 110% and 300% of the distance between the two fixation zones. This allows the flexible wall to bend and to be deployed (unfurled, unfolded) to increase the section of one of the lumens (10, 20) to the insertion section IS without increasing the dimension of the flexible wall. In the undeformed configuration of the flexible wall, the flexible wall is thus loosened. In the loosen state, the flexible wall may be folded, arc-shaped because of the gravity or disposed irregularly such as wavy (for example as in figure 8) because of the internal forces of the outer wall 102.

In a third embodiment of the physically separated configuration, the deformable wall 105 is an elastic wall as illustrated in figure 9. The elastic wall comprises resilient material such as elastomer so that the elastic wall recovers a unique undeformed shape when the external force disappears. The elastic wall comprises two opposite sides (105c, 105d), preferably, the longitudinal sides extending parallelly to the catheter axis A. The opposite sides (105c, 105d) are fixed to the outer wall 102 of the catheter body along a fixation zone. In one embodiment, the two fixation sides (105c, 105d) are fixed along the same fixation zone as represented in figure 11. The two fixation sides (105c, 105d) thus coincide. The elastic wall may also be manufactured in one piece with the outer wall 102 of the catheter body as represented in figure 10. The fixation zone is therefore the contact zone between the outer wall 102 and the elastic wall. Thanks to its resilient behavior, the elastic wall may have a dimension measured perpendicular to the catheter axis, i.e. between the two opposite sides (105c, 105d) which is comparable to the distance between the two fixation zones, for example ranging between 95% and 110% of the distance between the two fixation zones. The insertion of the tool (50, 60) applies a force to the elastic wall that stretches or elongates the elastic wall therefore providing, to the elastic wall, a dimension measured perpendicular to the catheter axis which larger than the distance between the two fixation zones, for example ranging between 110% and 300% of the distance between the two fixation zones.

The invention also relates to a method for assembling a catheter system, preferably for endoscopy of small orifices of a subject. The catheter system comprises the catheter 100 in the physically separated configuration and at least two tools (50, 60), each tool (50, 60) comprising a head part 51 with a head section HS.

The natural section NS of the first lumen 10 is smaller than the head section HS of the tool 50 intended to pass in said first lumen 10. Therefore, as explained above, the first lumen 10 is configured to reach a tool insertion state.

In the method, the head part 51 of each tool is sequentially inserted in one of the lumens (10, 20) through the input side of the catheter body. Only one tool is received in each lumen. In other words, one lumen receives either no tool or one tool. During the insertion, the deformable wall 105 is deformed by the head section HS of the tool 50 so that the first lumen 10 locally adopts, at the position of the head part 51 along the catheter axis A, the tool insertion state. In other words, at the position of the head part 51, the section of the first lumen 10 is increased, up to the insertion section IS. Consequently, the section of the second lumen 20 is proportionally reduced down to the reduced section RS at the position of the head part 51.

Each tool (50, 60) is slid along its corresponding lumen until the head part of the tools (50, 60) outputs from the output side of the catheter body as in figure 6. During the sliding of each tool, the position of the insertion part IP associated to said tool is shifted along the catheter axis A. One tool 50 may be slid up to the output side before the sliding of the second tool 60. Alternatively, the two tools (50, 60) may be simultaneously slid towards the output side. Because of the deformation of the lumen comprising the tool, let's say the first tool 50, which is positioned the most distally (i.e., which is inserted first), the other tool (the second tool 60) positioned more proximally is blocked in its progression by the first tool because of the deformation of the lumen around the first tool. Therefore, the second tool 60 cannot be slid before the first tool 50. This could also help in the insertion of the first tool if the second tool is more pushable than the first tool.

When the head part of each tool has completely exited the lumen, the adjacent lumens recover their natural sections NS along their whole length. The adjacent lumens thus recover their rest states.

When the tools (50, 60) are supported in their lumen (10, 20), the tool axis 53 of each tool is preferably substantially parallel to the catheter axis A.

### Merged configuration of the catheter 100

A second configuration of the catheter 100, also named the merged configuration, is illustrated in figure 12 and figure 13. In this second configuration, the first lumen 10 and the second lumen 20 are not physically separated by a deformable wall. This helps in reducing the size of the catheter 100, while keeping the functionalities. The first lumen 10 and the second lumen 20 are joined along an interfacing opening 106 thereby forming a merged lumen along the catheter axis A. The natural section of each lumen (10, 20) is thus delimited by the interfacing opening 106 which is a virtual area formed at the intersection of the two lumens (10, 20). Therefore, the two lumens (10, 20) do not overlap. The first lumen 10 and the second lumen 20 share the same merged volume and define a merged section which is the sum (and thus the union) of the natural section NS of the first lumen 10 and the natural section NS of the second lumen 20. The merged lumen has for instance a form of an eight or a snowman.

The second configuration allows to insert, in the merged lumen, a tool 50 having a head section HS larger than the natural section of first lumen 10. The tool 50 is thus inserted in both the first lumen 10 and at least partially in the second lumen 20 as shown in figure 14 wherein the first lumen 10 is defined by the shaded area whereas the second lumen 20 is defined by the dotted area. The insertion part IP of the first lumen 10 is thus defined by the position of the head part of the tool 50 along the catheter axis A. The insertion section IS comprises part of the merged section. More precisely, the insertion section IS is equal to the sum of the natural section NS of the first lumen 10 and the section of the second lumen 20 occupied by the head section HS. The section of the second lumen 20, at the position of the head part 51 of the tool 50, which is not occupied by the head part 51 is the reduced section RS. The reduced section RS is thus smaller than the natural section NS but does not result from a deformation of the catheter 100. More precisely, the reduced section RS is the natural section NS reduced by the volume of the head part 51 of the tool 50 disposed in the second lumen 20.

The invention also relates to a method for assembling a catheter system. The assembled catheter may be used for endoscopy of small orifices of a subject. The catheter system comprises the catheter 100 in the merged configuration and at least two tools (50, 60), each tool (50, 60) comprising a head part 51 with a head section HS.

The natural section NS of the first lumen 10 is smaller than the head section HS of the tool 50 intended to pass in said first lumen 10. Therefore, as explained above, the first lumen 10 is configured to reach a tool insertion state wherein the head part 51 of said tool 50 is inserted in the natural section of the lumen 10 and partially in the natural section of the adjacent lumen 20. The insertion part IP is thus located at the position of the head part 51.

In the method, the head part of each tool is sequentially inserted in one of the lumens (10, 20) through the input side of the catheter body.

During the insertion, the head part 51 occupies the first lumen 10 but also part of the second lumen 20. The first lumen 10 thus locally adopts, at the position of the head part 51 along the catheter axis A, the tool insertion state. In other words, at the position of the head part 51, the section occupied by the head part 51 is larger than the natural section of the first lumen 10. The insertion part IP thus has an insertion section IS larger than the natural section NS. Consequently, the section of the second lumen 20 is proportionally reduced down to the reduced section RS at the position of the head part 51.

Each tool (50, 60) is slid along its corresponding lumen until the head part of the tools (50, 60) outputs from the output side of the catheter body. During the sliding of each tool, the position of the insertion part IP associated to said tool is shifted along the catheter axis A. One tool 50 may be slid until output before the sliding of the second tool 60. Alternatively, the two tools (50, 60) may be simultaneously slid towards the output side. Because of the disposition of the head part 51 of the tool 50 both in the first and second lumen (10, 20), the other tool (the second tool 60) positioned more proximally compared to the first tool 50 cannot be slid before the first tool 50.

Each tool (50, 60), and more precisely its body part 52, is then placed in one of the lumens, each lumen receiving at most one tool. When the tools (50, 60) are supported in their lumen (10, 20), the tool axis 53 of each tool is preferably substantially parallel to the catheter axis A.

### EXAMPLE

The present invention is further illustrated by the following example of a medical system according to the invention. The medical system of this example is an endoscope that may be used for bronchoscopy.

The medical system comprises a medical device 200 having an operating channel of 3 mm and an external diameter of 5 mm.

The medical system further comprises a steerable catheter 100 having a circular section with an external diameter of 2.8 mm. The thickness of the braided outer wall 102 is constant at 0.25 mm.

The catheter 100 comprises two lumens: a first lumen 10 and a second lumen 20 adjacent to the first lumen 20. The first lumen 10 and the second lumen 20 are separated by an elastic wall 105. The elastic wall 105 has a distance between the two opposite sides (105c, 105d) of 2.1 mm. The elastic wall 105 has a thickness of 0.04 mm. The catheter 100 of this example is represented for illustration in figure 2.

The natural section NS of the first lumen 10 has a dome-shaped section with a height of 0.66 mm measured along the first axis 70. The natural section NS of the second lumen 20 has a dome-shaped section complementary to the first lumen 10 and therefore has a height of 1.6 mm.

The medical system further comprises two tools: a first tool 50 inserted in the first lumen 10 and a second tool 60 inserted in the second lumen 20.

The first tool 50 is an imaging device having a circular head section HS of 1 mm and a circular body section of 0.5 mm. The head section HS of the first tool 50 is thus larger than the natural section NS of the first lumen 10. Therefore, when the first tool 50 is inserted in the first lumen 10, the first lumen 10 reaches the tool insertion state wherein the insertion part IP following the position of the head part 51 has an insertion section IS having a height of 1 mm, the height of the insertion section IS being measured parallelly to the height of the dome-shaped natural section NS, i.e., in the first axis 70. Therefore, the elastic wall 105 is stretched by the head part 51 in the direction of the center of the second lumen 20 allowing to increase the height of the first lumen 10 from 0.66 mm to 1 mm. The insertion section IS thus has the shape of an ellipsoid. The height of the second lumen 20 is proportionally reduced from 1.63 mm to 1.26 mm. The reduced section RS of the second lumen 20 thus has the shape of a crescent.

The second tool 60 is a therapy tool having a generally circular head section HS of 1.8 mm and a circular body section of 1.5 mm. The head section HS of the second tool 60 is thus larger than the natural section NS of the second lumen 20. The second tool 60 is inserted after the head part 51 of the first tool 50 is completely output. When the second tool 60 is inserted in the second lumen 20, the second lumen 20 reaches the tool insertion state wherein the insertion part IP following the position of the head part of the second tool 60 has an insertion section IS having a height of 1.8 mm. Therefore, the elastic wall 105 is stretched by the head part in the direction of the center of the first lumen 10 allowing to increase the height of the second lumen 20 from 1.6 mm to 1.8 mm. The insertion section IS of the second lumen 20 thus has the shape of an ellipsoid. The height of the first lumen 10 is proportionally reduced from 0.66 mm to 0.46 mm. The reduced section RS of the first lumen 10 thus has the shape of a crescent.

The catheter 100 of this example thus allows to receive two tools wherein the sum of the section of the tools is larger than the internal diameter of the catheter without increasing the external diameter of the catheter. This allows to insert the tools when the catheter 100 is inserted in a medical device 200 with an internal diameter smaller than the sum of the section of the tools.

This allows to use an imaging device during the treatment so that to monitor in real time the treatment. Thanks to the reduced size of the catheter 100, the treatment may be performed in the periphery of a lung wherein the size of the bronchial tubes is small, i.e., as small as 2.8 mm. Indeed, the medical device 200 may be inserted in tubes down to a size of 5 mm. The catheter 100 is then slid in the medical device 200 and guided into tubes having a size down to 2.8 mm. The tools are then pushed along the same direction (since they are not guided) up to very small tubes. The size of the natural orifices that can be reached is limited by the sum of the body section of the treatment tool and the head section of the imaging device which are superposed in the orifice.

The size of the reached bronchial tubes is well below the size of the bronchial tubes that may be reached using a known multi-lumen catheter. Indeed, to insert the two tools (50, 60) described above, the known multi-lumen catheter must have an external diameter larger than 5 mm. It is thus impossible to push the non-guidable tools up to bronchial tubes of 2.8 mm because of the too many ramifications between the tubes of 5 mm and the tubes of 2.8 mm.

### REFERENCES

10, 20 - Lumen // 30 - Auxiliary lumen // 50, 60 - Tool // 51 - Head part // 52 - Body part // 53 - Tool axis // 70 - First axis along which the lumens are disposed // 75 - Second axis perpendicular to the first axis // 100 - Catheter // 101 - Distal end // 102 - Outer wall // 105 - Deformable wall // 105a - First side of the flap // 105b - Second side of the flap // 105c, 105d - Opposite sides of the deformable wall // 106 - interfacing opening // 200 - Medical device // A - Catheter axis // HS - Head section // IP - Insertion part // IS - Insertion section // NS - Natural section // RS - Reduced section

## Claims

1. A catheter (100) , said catheter (100) comprising a catheter body defining a catheter axis (A) and comprising at least a first lumen (10) and a second lumen (20) adjacent to the first lumen (10), each lumen (10, 20) extending along the catheter axis (A), each lumen (10, 20) having a natural section (NS), each lumen (10, 20) being configured to allow passage of a tool along the catheter axis (A), the tool comprising a head part (51) with a head section (HS),
wherein the natural section (NS) of the first lumen (10) is smaller than the head section (HS) of the tool intended to pass in said first lumen (10), the first lumen (10) being configured to reach a tool insertion state, the first lumen (10) comprising, in the tool insertion state, an insertion part (IP) configured to receive the head part (51) of said tool, the insertion part (IP) comprising the natural section (NS) of the first lumen (10) and at least part of a natural section (NS) of the second lumen (20), the insertion part (IP) extending locally along the catheter axis (A), the insertion part (IP) having a variable position along the catheter axis (A) to allow the passage of the head part (51) along the catheter axis (A).

2. The catheter (100) according to claim 1 wherein the catheter body is configured to be inserted into a channel of a medical device for:
- endoscopy of natural orifices, the endoscopy being preferably one of bronchoscopy, gastroscopy, gastro-enterology, colonoscopy, endoscopic retrograde cholangiopancreatography, urology, or ear, nose and throat endoscopy,
- laparoscopy, or
- cardiology.

3. The catheter (100) according to claim **1** or **2** wherein the catheter body has an external catheter sectional size lower than 5 mm, preferably ranging between 1.2 mm and 3 mm, and more preferably between 1.8 mm and 2.8 mm.

4. The catheter (100) according to any one of claims **1** to **3** wherein the adjacent lumens (10, 20) are superposed along a first axis (70) perpendicular to the catheter axis (A), at least one auxiliary lumen (30) being positioned along a second axis (75) perpendicular to the first axis (70) and perpendicular to the catheter axis (A).

5. The catheter (100) according to any one of claims **1** to **4**, wherein the first lumen (10) and the second lumen (20) are separated by a deformable wall (105), the first lumen (10) and the second lumen (20) comprising a locally variable section between the natural section (NS), a reduced section (RS) smaller than the natural section (NS) and an insertion section (IS) larger than the natural section (NS), the deformable wall (105) comprising an undeformed configuration and a deformed configuration, wherein the undeformed configuration defines the natural section (NS) of the first lumen (10) and the second lumen (20), wherein the insertion part (IP) of the first lumen (10) comprises the deformed configuration of the deformable wall (105), the first lumen (10) thereby having the insertion section (IS), the second lumen (20) having the reduced section (RS) at a position of the insertion part (IP) along the catheter axis (A).

6. The catheter (100) according to claim **5,** wherein the deformable wall (105) comprises at least one flap, each flap comprising a first side (105a) fixed to an outer wall (102) of the catheter body and a second side (105b) opposite the first side (105a), a deformation of the deformable wall (105) between the undeformed configuration and the deformed configuration being a variation of the position of the second side (105b) relatively to the outer wall (102) of the catheter body.

7. The catheter (100) according to claim **5,** wherein the deformable wall (105) is a flexible wall, the flexible wall comprising two opposite sides (105c, 105d), each opposite side being fixed to an outer wall (102) of the catheter body along a fixation zone, a dimension of flexible wall between the two opposite sides (105c, 105d) in the undeformed configuration being larger than a minimum distance between the two fixation zones leading to a loosen wall, the flexible wall being unfolded in the deformed configuration.

8. The catheter (100) according to claim **5,** wherein the deformable wall (105) is an elastic wall, the elastic wall comprising two fixation sides (105c, 105d), each opposite side being fixed to an outer wall (102) of the catheter body along a fixation zone, a dimension of elastic wall between the two fixation sides (105c, 105d) in the undeformed configuration being equal to a minimum distance between the two fixation zones, the elastic wall being stretched in the deformed configuration.

9. The catheter (100) according to any one of claims **5** to **8**, wherein the deformable wall (105) is made of metal or polymer, preferably thermoplastics, elastomers, thermosets, thermoplastic elastomer, polyamide or fluoropolymer.

10. The catheter (100) according to any one of claims **1** to **4**, wherein the first lumen (10) and the second lumen (20) are joined along an interfacing opening (106) thereby forming a merged lumen along the catheter axis (A), a union of the natural section (NS) of the first lumen (10) and the natural section (NS) of the second lumen (20) forming a merged section, the insertion part (IP) of the first lumen (10) defining an insertion section (IS) comprising at least part of the merged section larger than the natural section (NS) of said first lumen (10), the head part (51) passing in the first lumen (10) and the second lumen (20).

11. A medical system comprising:
- A medical device (200) comprising an operating channel;
- a catheter (100) comprising a catheter body defining a catheter axis (A) and comprising at least a first lumen (10) and a second lumen (20) adjacent to the first lumen (10), each lumen (10, 20) extending along the catheter axis (A), each lumen (10, 20) having a natural section (NS), each lumen (10, 20) being configured for passing a tool along the catheter axis (A), the catheter body being inserted into the operating channel of the medical device (200); and
- at least one tool, each tool (50, 60) comprising a head part (51) with a head section (HS), each tool (50, 60) being received in one of the lumens (10, 20) of the catheter body;
wherein the head section (HS) of a first tool (50) of the at least one tool is larger than the natural section (NS) of the first lumen (10), the first lumen (10) being configured to reach a tool insertion state, the first lumen (10) comprising, in the tool insertion state, an insertion part (IP) configured to receive the head part (51) of the first tool (50), the insertion part (IP) comprising the natural section (NS) of the first lumen (10) and at least part of a natural section (NS) of the second lumen (20), the insertion part (IP) extending locally along the catheter axis (A), the insertion part (IP) having a variable position along the catheter axis (A) to allow the passage of the head part (51) of the first tool (50) along the catheter axis (A).

12. The medical system according to claim **11** wherein a first tool (50) of the at least one tool further comprises a body part (52) having a body section, the body section being smaller than or equal to the natural section (NS) of the first lumen (10) and wherein the head section (HS) of a second tool (60) of the at least one tool (50, 60) is larger than the natural section (NS) of the second lumen (20), the second lumen (20) being configured to reach a tool insertion state, the second lumen (20) comprising, in the tool insertion state, an insertion part (IP) configured to receive the head part (51) of the second tool (60), the insertion part (IP) comprising the natural section (NS) of the second lumen (20) and at least part of a natural section (NS) of the first lumen (10), the insertion part (IP) extending locally along the catheter axis (A), the insertion part (IP) having a variable position along the catheter axis (A) to allow the passage of the head part (51) along of the second tool (60) the catheter axis (A) following the passage of the first tool (50).

13. The medical system according to claim **11** or **12** wherein at least one of the tools (50, 60) is an imaging device, the medical system thereby forming and endoscope.

14. A method for assembling a catheter system, the catheter system comprising a catheter (100), said catheter (100) comprising a catheter body defining a catheter axis (A) and comprising at least a first lumen (10) and a second lumen (20) adjacent to the first lumen (10), each lumen (10, 20) extending along the catheter axis (A), each lumen (10, 20) having a natural section (NS), each lumen (10, 20) being configured for passing a tool along the catheter axis (A), wherein the first lumen (10) and the second lumen (20) are separated by a deformable wall (105), each of the first lumen (10) and the second lumen (20) comprising a locally variable section between the natural section (NS), a reduced section (RS) smaller than the natural section (NS) and an insertion section (IS) larger than the natural section (NS), wherein the deformable wall (105) comprises an undeformed configuration and a deformed configuration, wherein the undeformed configuration defines the natural section (NS) of the first lumen (10) and the second lumen (20), the catheter system further comprising at least two tools (50, 60), each tool (50, 60) comprising a head part (51) with a head section (HS), the natural section (NS) of the first lumen (10) being smaller than the head section (HS) of the tool intended to pass in said first lumen (10), said first lumen (10) being configured to reach a tool insertion state, the catheter body comprising an input side and an output side,
the method comprising:
- inserting sequentially the head part (51) of each tool (50, 60) in one of the lumens (10, 20) through the input side, each lumen (10, 20) receiving at most one tool, the deformable wall (105) of the first lumen (10) being deformed to locally adopt, at a position of the head part (51) along the catheter axis (A), the tool insertion state by increasing, at the position of the head part (51) along the catheter axis (A), a section of the first lumen (10) up to the insertion section (IS) comprising the natural section (NS) of the first lumen (10) and at least part of a natural section (NS) of the second lumen (20) thereby forming an insertion part (IP) extending locally along the catheter axis (A), a section of the second lumen (20) being proportionally reduced down to the reduced section (RS) at the position of the head part (51) along the catheter axis (A);
- sliding each tool (50, 60) along the lumens (10, 20) until the head part (51) of the tools (50, 60) outputs from the output side, thereby shifting a position of the insertion part (IP) along the catheter axis (A).

15. A method for assembling a catheter system, the catheter system comprising a catheter (100), said catheter (100) comprising a catheter body defining a catheter axis (A) and comprising at least a first lumen (10) and a second lumen (20)s adjacent to the first lumen (10), each lumen (10, 20) extending along the catheter axis (A), each lumen (10, 20) having a natural section (NS), each lumen (10, 20) being configured for the passage of a tool, wherein the first lumen (10) and the second lumen (20) are joined along an interfacing opening (106) thereby forming a merged lumen along the catheter axis (A), the union of the natural section (NS) of the first lumen (10) and the natural section (NS) of the second lumen (20) forming a merged section, the catheter system further comprising at least two tools (50, 60), each tool (50, 60) comprising a head part (51) having a head section (HS), the head section (HS) of a first tool (50) of said tools being larger than the natural section (NS) of the first lumen (10), the first lumen (10) being configured to reach a tool insertion state, the first lumen (10) comprising, in the tool insertion state, an insertion part (IP) configured to receive the head part (51) of the tool, the insertion part (IP) defining an insertion section (IS) comprising at least part of the merged section larger than the natural section (NS), the catheter body comprising an input side and an output side,
the method comprising:
- inserting sequentially the head part (51) of each tool (50, 60) in the catheter body through the input side, the head part (51) of the first tool (50) being inserted in the merged lumen, the first lumen (10) thereby reaching the tool insertion state with the insertion part (IP) located at the location of the head part (51), the insertion section (IS) being equal to a sum of the natural section (NS) of the first lumen (10) and a part of the natural section (NS) of the second lumen (20) occupied by the head section (HS);
- sliding each tool (50, 60) along the catheter axis (A) until the head part (51) of each tool (50, 60) completely outputs from the output side, thereby shifting the position of the insertion part (IP) along the catheter axis (A);
- placing each tool (50, 60) in one of the lumens (10, 20), each lumen (10, 20) receiving at most one tool.
